# EUROPÄISCHE PATENTSCHRIFT

(11) **EP 1 421 541 B1**
(45) Veröffentlichungstag und Bekanntmachung des Hinweises auf die Patenterteilung: **06.11.2013**
(21) Anmeldenummer: 02758427.5
(22) Anmeldetag: 02.08.2002
(51) Int. Cl.: G06F 19/00

(54) **HÄMODIALYSE- UND/ODER -FILTRATIONSGERÄT MIT AUTOMATISIERTER DATENAKTUALISIERUNG**
HEMODIALYSIS- AND/OR -FILTRATION DEVICE WITH AUTOMATED DATA ACTUALISATION
APPAREIL D'HÉMODIALYSE- ET/OU -FILTRATION À ACTUALISATION AUTOMATISÉE DES DONNÉES

(30) Priorität: 30.08.2001 DE 10142516
(43) Veröffentlichungstag der Anmeldung: 26.05.2004
(73) Patentinhaber: Fresenius Medical Care Deutschland GmbH, 61352 Bad Homburg (DE)
(72) Erfinder: STAHL, Thomas, 97209 Veitshöchheim (DE); MENNINGER, Stefan, 97076 Würzburg (DE); PFEUFFER, Matthias, 97714 Ebenhausen (DE)
(74) Vertreter: Laufhütte, Dieter
(86) Internationale Anmeldenummer: PCT/EP2002/008650
(87) Internationale Veröffentlichungsnummer: WO 2003/020332

(56) Entgegenhaltungen:
- EP-A- 1 128 279
- WO-A-99/64966
- US-A- 5 710 922
- US-A- 5 842 173
- US-A- 5 899 998

## Beschreibung

Die Erfindung betrifft ein Hämodialyse- und/oder -filtrationsgerät mit mindestens zwei Rechnereinheiten nach dem Oberbegriff des Anspruchs 1.

In der Medizintechnik sind mikroprozessorgesteuerte Geräte in vielen Anwendungsbereichen zu finden. Die Entwicklung hat dabei zur Lösung immer komplexerer Aufgaben entsprechend komplexe Systeme hervorgebracht, die selten durch nur ein Mikroprozessorsystem effizient gesteuert und überwacht werden können.

In der Medizintechnik ist es aufgrund von Sicherheitsüberlegungen verbreitet, neben einer mikroprozessorgesteuerten Steuereinheit eine ebenso gesteuerte Überwachungseinheit zu stellen. Andererseits ist es oft auch ohne Überwachungseinheiten erforderlich, verschiedene Funktionen auf mehrere Geräte bzw. deren Rechnereinheiten zu verteilen und diese parallel arbeiten zu lassen, wobei sie nach außen wie ein komplexes Gesamtgerät wirken.

Die DE 198 49 787 C1 beschreibt z.B. ein Blutbehandlungsgerät, das aus mehreren Steuereinheiten besteht. Mindestens zwei der Steuereinheiten weisen je einen Aktionsrechner und einen Hilfsrechner auf. Alle Rechner sind durch ein bestimmtes Kommunikationssystem miteinander verbunden. Das Gerät erlangt durch diesen Aufbau und durch bestimmte Fehlerbehandlungsroutinen ein flexibles Verhalten im Fehlerfall, bei der jede Steuereinheit selbstständig agieren kann, sowie eine effiziente Durchführung der Blutbehandlung selbst, da die Aufgaben des Gerätes auf verschiedene Steuereinheiten verteilt werden.

Bei einem solchen multiprozessoralen System müssen zwangsläufig regelmäßig Daten zwischen den verschiedenen Rechnereinheiten ausgetauscht werden, damit alle Einheiten über den gleichen Datenbestand verfügen. Dazu ist es naheliegend, Daten entweder per individueller Anfrage einer Rechnereinheit von einer anderen Rechnereinheit zur Verfügung zu stellen oder gezielt von einer Rechnereinheit zu versenden, wenn das Programm dies vorsieht. Bei wachsender Komplexität der Software für jede Steuer- bzw. Rechnereinheit wird ein solches Verfahren jedoch schnell uneffizient und für den Programmierer zunehmend unübersichtlich. Er muß an jeder Stelle eines Programmes für eine Rechnereinheit den Überblick behalten, welche Datenelemente auf welche Weise kommuniziert werden müssen, damit die jeweilige Rechnereinheit über die aktuellen Daten verfügt. Bei jeder erforderlichen Kommunikation müssen neue Befehle in den Programmcode eingefügt werden. Da es bei der komplexen Softwarepflege durchaus üblich ist, daß die Software verschiedener Rechnereinheiten von verschiedenen Programmierern gepflegt wird, Bedarf es zudem einer aufwendigen Abstimmung, die leicht Anlaß zu Fehlern geben kann.

Auch die Bereitstellung aller im System zur Verfügung stehenden Daten in zentralen Einheiten, wie sie durch z.B. in der DE 37 36 712 A1. beschriebenen Art und Weise eingesetzt werden könnten, ist nachteilig. Falls es zum Ausfall dieser zentralen Einheiten kommt, sind gleichzeitig alle Rechnereinheiten der Möglichkeit beraubt, über die aktuellen Systemparameter zu verfügen. Zusätzlich sind auch hier individuelle Schreib- und Lesebefehle im Bedarfsfall erforderlich.

Der Erfindung liegt die Aufgabe zugrunde, ein gattungsgemäßes medizinisches Gerät derart weiterzubilden, daß ein automatisierter Vorgang ständig für die Aktualisierung der in einer Rechnereinheit benötigten Datenelemente sorgt und gleichzeitig eine vereinfachte Entwicklung und Pflege der Software der Rechnereinheiten möglich ist, bei der während der Programmierung weitgehend auf individuelle Schreibbefehle und/oder Anforderungsbefehle zur Aktualisierung der Daten verzichtet werden kann.

Nach der Lehre der Erfindung wird diese Aufgabe durch ein Hämodialyse- und/oder -filtrationsgerät nach dem Anspruch 1 gelöst.

Mit Hilfe des erfindungsgemäßen Gerätes ist es möglich, daß der Programmierer während des Programmierens jedes Datenelement automatisch so verwenden kann, als ob es immer den aktuellen Wert innehat. Besondere Anforderungen oder Abstimmungen mit anderen Rechnereinheiten sind auf ein Minimum reduziert. Es müssen lediglich gewisse Basisstrukturen einmalig bereitgestellt werden.

Vorteilhaft kann dabei zweckmäßigerweise jedem Datenelement - und insbesondere den zum Datenaustausch bestimmten - eine für die Rechnereinheiten global eindeutige Identifizierungsinformation zugeordnet sein.

In einer weiteren vorteilhaften Ausführungsform wird jedem zum Datenaustausch bestimmten Datenelement ein zweites Datenelement zugeordnet ist, das Informationen darüber beinhaltet, an welche Rechnereinheit aktualisierte Datenelemente automatisch übertragen werden sollen. Hierzu braucht gemäß einer Weiterentwicklung der Ausführungsform von der sendenden Rechnereinheit zunächst nur Speicherplatz bereitgestellt zu werden. Die Inhalte dieser zweiten Datenelemente brauchen erst während eines Initialisierungsprozesses des medizinischen Gerätes mit Inhalten beschrieben zu werden, was durch einmalig ablaufende Anweisungen gewährleistet werden kann.

Das Kommunikationsmedium zwischen den Steuereinheiten kann eine drahtlose Verbindung darstellen (z.B. RF, Infrarot) oder aber eine Kabelverbindung, für die dem Fachmann eine Vielzahl von Alternativen zur Verfügung stehen.

Als Kommunikationsschnittstelle wird bevorzugt eine CAN-Bus Schnittstelle eingesetzt. Prinzipiell ist aber auch jede andere Schnittstellenanbindung oder eine Kombination davon möglich (RS 232, ...).

Bei einer Ausführungsform der Erfindung kann die Überprüfung bei der Wertzuweisung des ersten Datenelementes so gestaltet sein, daß im positiven Falle durch die Wertzuweisung eine Übertragungsliste um dieses Datenelement und der Adressateninformation erweitert wird. In regelmäßigen Abständen wird diese Liste gesendet,

In einer anderen Ausführungsform der Erfindung ist das Programm der jeweils versendenden Rechnereinheit so gestaltet, daß ein Programmbereich regelmäßig in ausreichend kleinen Abständen aufgerufen wird, der zunächst den Status der einzelnen Datenelemente überprüft. Eine solche Überprüfung hat zum Gegenstand festzustellen, ob sich die zur Versendung in Frage kommenden ersten Datenelemente gegenüber der vorangegangen Überprüfung überhaupt verändert haben, so daß eine erneute Versendung notwendig ist. Auch dies wird als eine Überprüfung der ersten Datenelemente bei einer Wertzuweisung verstanden.

Für diese Überprüfung können den ersten Datenelementen dritte Datenelemente zugeordnet sein, die die Daten der ersten Elemente im vorangegangenen Zyklus beinhalten, damit die Überprüfung anhand eines Vergleiches der ersten und dritten Datenelemente durchgeführt werden kann. Es können andererseits auch dritte Datenelemente dergestalt den ersten Datenelementen zugeordnet sein, die bei einer anstehenden Übertragung einen ersten Zustand und bei keiner anstehenden Übertragung einen zweiten Zustand aufweisen. Der erste Zustand kann automatisch dann zugewiesen werden, wenn dem entsprechendem ersten Datenelement irgendein Wert zugewiesen wird - unabhängig davon, ob er sich geändert hat. Falls keine Zuweisung erfolgt, verbleibt das dritte Datenelement im zweiten Zustand, in den es nach der letzten Versendung automatisch versetzt wurde.

Zum Schreiben neuer Werte in die Datenelemente kann man sich zweckmäßigerweise bereits vorhandener Strukturen in bestimmten, vor allem objektorientierten Programmiersprachen bedienen. Besonders vorteilhaft ist die Verwendung von für jeden Datentyp spezifischen Zuordnungsfunktionen, wie sie z.B. die Sprache C bereitstellt. Mit Hilfe der Zuordnungsfunktion kann gleichzeitig neben der Zuweisung die Überprüfung der Bereitstellung zur Versendung standardisiert und einfach implementiert werden.

Ausführungsformen für ein Blutbehandlungsgerät dieser Art sind in der DE 198 49 787 C1 beschrieben, auf deren Offenbarung hiermit ausdrücklich Bezug genommen wird. Eine Ausführungsform eines erfindungsgemäßen Hämodialyse- und/oder -filtrationsgerätes kann eine Bedieneinheit als erste Steuereinheit, eine Hydraulikeinheit als zweite Steuereinheit und eine Funktionseinheit als dritte Steuereinheit umfassen, wobei alle Steuereinheiten je einen Aktionsrechner und einen Hilfsrechner beinhalten, die je ein Programm gemäß des kennzeichnenden Teils nach Anspruch 1 zur gegenseitigen Aktualierung ihrer Daten aufweisen.

Weitere Einzelheiten und Vorteile der Erfindung werden anhand von in den Zeichnungen dargestellten Ausführungsbeispielen näher beschrieben. Es zeigen:
- Fig. 1: einen objektorientierten Programmablaufplan einer ersten Ausführungsform eines Mikroprozessorprogrammes für ein erfindungsgemäßes medizinisches Gerät,
- Fig. 2: ein Blockschaltbild eines Hämodialysegerätes als Blutbehandlungsgerät mit Rechnereinheiten gemäß einer zweiten Ausführungsform der Erfindung und
- Fig. 3: ein Blockschaltbild eines Ausschnitts der beteiligten Speichereinheiten des Gerätes nach Fig. 2, in denen die ersten, zweiten und dritten Datenelemente der jeweiligen Rechnereinheit abgelegt werden.

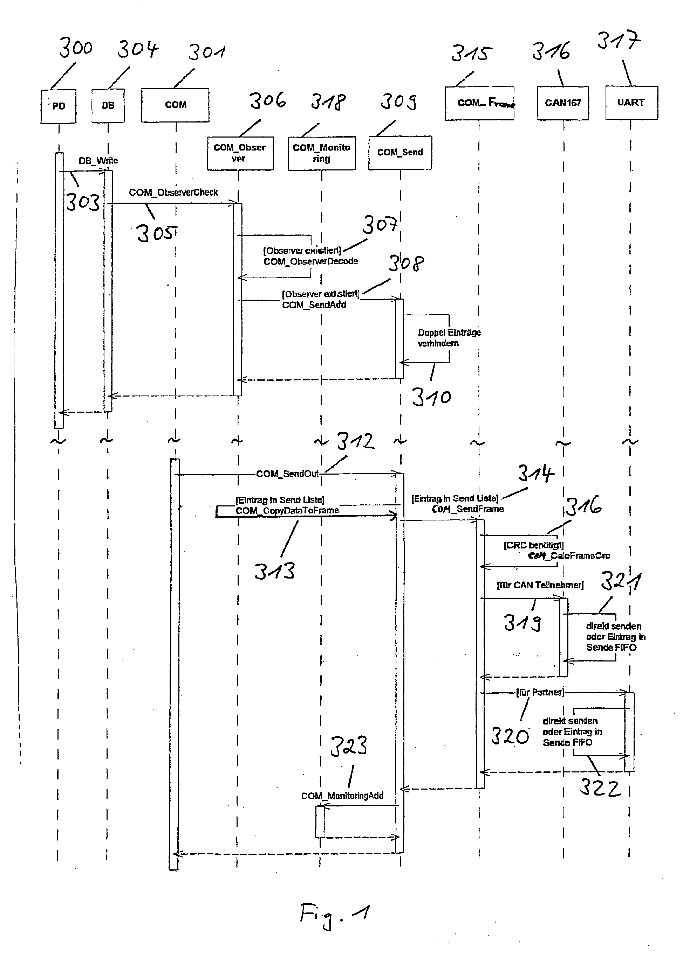

Die Fig. 1 gibt einen objektorientierten Programmablauf wieder, der den Ablauf einer ersten Ausführungsform eines Mikroprozessorprogrammes auf einer ersten Rechnereinheit eines erfindungsgemäßen medizinischen Gerätes widerspiegelt. Die Kästchen am oberen Bildrand repräsentieren verschiedene Objekte. Die gestrichelten Linien von den Objekten nach unten stellen Zeitachsen dar, wobei eine waagerechte Linie einen für alle Objekte gemeinsamen Zeitpunkt symbolisiert. Die langen Kästchen auf den Zeitachsen kennzeichnen die Zeitbereiche, während deren ein Objekt aktiv ist. Die waagerechten Pfeile betreffen Aktionen, welche von einem Objekt initiiert und von einem anderen oder vom gleichen Objekt umgesetzt werden. Ausdrücke in eckigen Klammern betreffen Bedingungen, d.h. die darunter beschriebene Aktion wird nur im des Erfüllens dieser Bedingung durchgeführt.

Die Zeitachsen lassen sich in zwei Abschnitte einteilen: Einen Abschnitt zum Ablauf von Prozessobjekten 300 und einen Abschnitt zum Ablauf die Sendesteuerobjektes 301. Dabei sorgt das erfindungsgemäße Programm dafür, daß das Sendesteuerobjekt 301 in regelmäßigen Abständen aufgerufen wird. Dazwischen können beliebig viele und verschiedene Projektobjekte 300 aufgerufen werden.

Bei der Abarbeitung eines Prozeßobjekts 300 kann es zu einer Zuweisung eines Wertes zu einem ersten Datenelement kommen. In diesem Fall wird ein Schreibbefehl 303 initiiert, der zum Eintrag des neuen Wertes in eine lokale Datenbank führt. Der Eintrag in die Datenbank wird durch das Datenbankobjekt 304 durchgeführt, das auch die weitere Überprüfung einleitet, ob der Wert an andere Rechnereinheiten übertragen werden soll. Ein Eintrag in die Datenbank kann bei jeder Zuweisung oder alternativ auch nur bei Zuweisung eines geänderten Wertes erfolgen.

Über einen Objektaufruf 305 wird nun das Überprüfungsobjekt 306 aufgerufen. Durch eine Abfrage 307 werden den ersten Datenelementen zugeordnete zweite Datenelemente dahingehend überprüft, ob eine Übersendung der (neuen) Werte der ersten Datenelemente an eine weitere Rechnereinheit vorgesehen ist. Falls diese Bedingung erfüllt ist, wird durch den Objektaufruf 308 durch ein Listenvorbereitungsobjekt 309 ein Eintrag des ersten Datenelementes sowie ggf. der Informationen zu den Zielrechnereinheiten vorgenommen. Alternativ kann auch nur das erste Datenelement ansich eingetragen werden, wobei die Zielrechnerinformationen erst bei der Übersendung von der lokalen Datenbank eingelesen wird. Durch eine Prüfprozedur 310 werden Doppeleinträge verhindert.

Nach dem Durchlaufen der Projektobjekte 300 wird in regelmäßigen Abständen das Sendesteuerobjekt 301 aufgerufen. Genausogut kann es möglich sein, das Sendesteuerobjekt in definierten Einzelfällen gezielt aufzurufen. Der Ausdruck "regelmäßige Abstände" soll dabei dergestalt interpretiert werden, daß das Sendesteuerobjekt in ausreichend kurzen Abständen die Inhalte geänderter erster Datenelemente so schnell weiterleitet, wie es für die korrekte technische Funktion der anderen, empfangenden Rechnereinheiten notwendig ist.

Das Sendesteuerobjekt 301 ruft seinerseits das Listenvorbereitungsobjekt 309 durch den Aufruf 312 auf. Dort folgt die Abfrage 313, ob sich Einträge in der Sendeliste befinden. Im positiven Fall wird eine Übersendungsdatenpaket ("Frame") erstellt. Dieses wird dann durch die Anweisung 314 an das Datenübersendungsobjekt 315 übertragen. Dort wird ggf. eine CRC-Sicherung vorgenommen (Abfrage/Anweisung 316).

Anschließend werden die Daten durch die einzelnen Kommunikationsschnittstellen versendet. In Fig. 1 wird dies einerseits durch das CAN-Schnittstellenobjekt 316 und andererseits durch das serielle Schnittstellenobjekt (RS 232) 317 durchgeführt, die durch die bedingten Aufrufe 319 und 320 gestartet werden. Dabei kann vorgesehen sein, die Daten direkt zu senden oder in einen Puffer, hier beispielsweise vom Typ FIFO, für die eigentliche Übersendung zu sammeln (Anweisungen 321 und 322). Zum Abschluß kann durch ein Protokollobjekt 318 eine Protokollierung der Sendevorgänge erfolgen (Anweisung 323). Damit ist das Sendesteuerobjekt 301 zum Ende gelangt. Der Empfang der Datenpakete durch die empfangenden Rechnereinheiten geschieht in an sich bekannter Weise, wobei eine Rückbestätigung an die sendende Rechnereinheit erfolgen kann.

Durch diese Gestaltung des Mikroprozessorprogrammes der sendenden Rechnereinheit ist es möglich, eine standardisierte und effektive Aktualisierung von Parameterwerten des medizinischen Gerätes sicherzustellen, die von verschiedenen Rechnereinheiten verwendet werden, ohne daß die empfangenden Rechnereinheiten solche Werte individuell anfordern müssen.

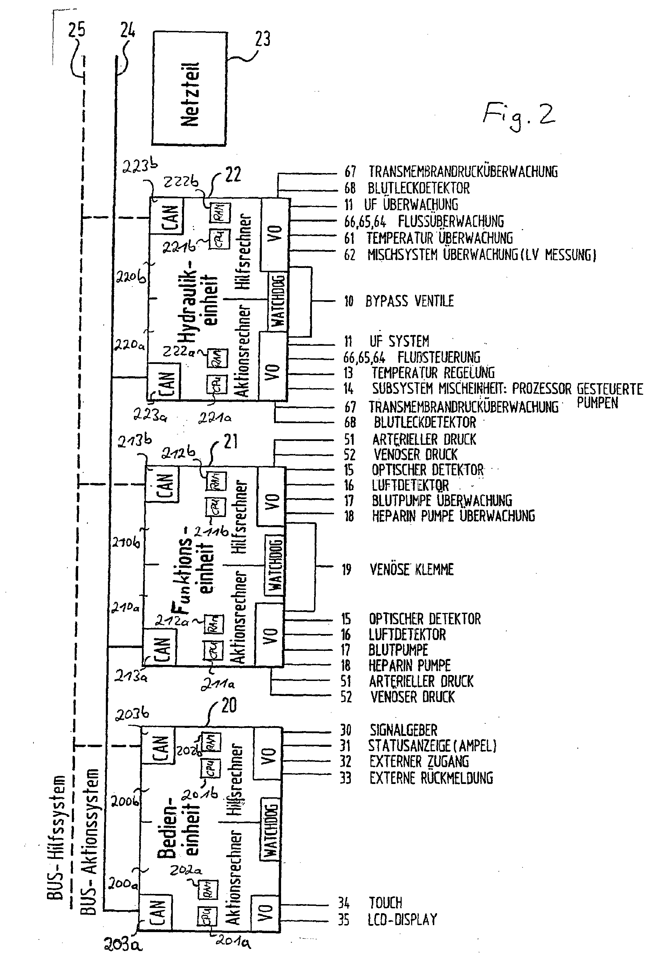

Die Erfindung wird nun anhand eines Hämodialysegerätes als Beispiel für ein erfindungsgemäßen medizinischen Gerätes näher erläutert. In Fig. 2 ist schematisiert der Aufbau eines Hämodialysegerätes dargestellt, wie er im wesentlichen auch schon Gegenstand der DE 198 49 787 C1 ist. Für die dort verwendeten Bezugszeichen wurden hier übereinstimmende Bezugszeichen gewählt. Zusätzliche Teile, die zum Verständnis der Erfindung hilfreich sind, tragen neue Bezugszeichen.

Das Hämodialysegerät nach Fig. 2 weist drei Steuereinheiten auf: eine Bedieneinheit 20, eine Funktionseinheit 21 und eine Hydraulikeinheit 22. Die einzelnen Einheiten bestehen jeweils aus einem Aktionsrechner 200a, 210a bzw. 220a und einem Hilfsrechner 200b, 210b bzw. 220b. Die Aktionsrechner jeder Steuereinheit sind über einen Aktionsbus 24 und die Hilfsrechner über einen Hilfsbus 25 miteinander verbunden, die in diesem Beispiel beide als CAN-Bus ausgebildet sind. Zur Kommunikation zwischen den Aktionsrechnem verschiedener Steuereinheiten wird seitens des sendenden Aktionsrechners über seine als CAN-Schnittstelle ausgebildete Kommunikationsschnittstelle 203a, 213a bzw. 223a eine adressierte Nachricht an den Aktionsbus 24 gelegt, die aufgrund der Adressierung zu dem jeweiligen als Empfänger dienenden Aktionsrechner gelangt. Analoges gilt für die Kommunikation der Hilfsrechner über ihre als CAN-Interface ausgebildete Kommunikationsschnittstelle 203b, 213b bzw. 223b. Weiterhin ist eine Kommunikation innerhalb jeder Steuereinheit zwischen einem Aktionsrechner und einem Hilfsrechner möglich (nicht näher dargestellt), so daß Nachrichten auch von einem Aktionsrechner einer Steuereinheit zu einem Hilfsrechner einer anderen Steuereinheit bzw. umgekehrt gesendet werden können. Bei der direkten Verbindung innerhalb einer Steuereinheit kann es sich um ein zweites Bussystem handeln.

Die Aktionsrechner weisen des weiteren einen Mikroprozessor 201a, 211a bzw. 221a und eine Speichereinheit 202a, 212a bzw. 222a auf. Analoges gilt für die Hilfsrechner mit Mikroprozessoren 201 b, 221b bzw. 221b und Speichereinheiten 202b, 212b bzw. 222b.

An die Aktions- und Hilfsrechner sind über eine geeignete Schnittstelle ("VO") die Aktoren und Sensoren des medizinischen Gerätes angeschlossen. Da deren Funktion zur Erläuterung der Erfindung von untergeordneter Bedeutung ist, wird diesbezüglich auf die DE 198 49 787 C1 verwiesen.

Alle Elemente eines Rechners bzw. einer Rechnereinheit sind intern zur Kommunikation miteinander verbunden. Auf die Darstellung dieser dem Fachmann geläufigen Kommunikationsverbindung wurde der Übersichtlichkeit halber verzichtet.

Das Programm, das erfindungsgemäß in den Speicherelementen der Rechner zur Ausführung im jeweiligen Mikroprozessor untergebracht ist, geht nun wie folgt von

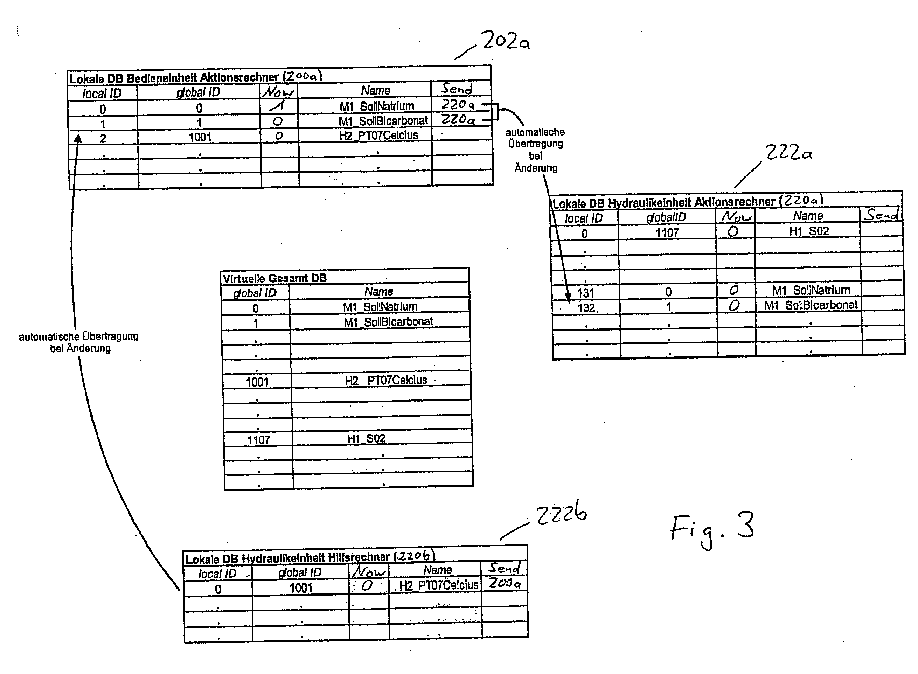

In jedem Speicherelement ist ein Bereich abgetrennt, der fortan als lokale Datenbank ("Lokale DB") bezeichnet wird. Diese lokalen Datenbanken können gemäß der schematisch in Fig. 3 gezeigten Ausführungsform aufgebaut sein. Jede lokale Datenbank beinhaltet alle Datenelemente ("Name"), die von dem jeweiligen Rechner verwendet werden. Diese Datenelemente stellen die ersten Datenelemente dar. Diesen ersten Datenelementen sind Informationen zugeordnet, die sie global - also in Zusammenschau mit allen anderen Datenelementen der anderen Rechner - eindeutig identifizierbar machen ("global ID"). Sollten eindeutige Namen für die ersten Datenelemente verwendet werden - wie in Fig. 3 - ist eine Extra-Zuordnung zur Identifikation nicht zwingend erforderlich. Eine eindeutige Nummerierung aller ersten Datenelemente erleichtert jedoch die Implementierung des erfindungsgemäßen Programmes.

Es kann des weiteren vorgesehen sein, lokal den ersten Datenelementen eine eigene lokale Identifikationsinformation zuzuordnen ("local ID"). Damit existieren ja nach Zahl der ersten Datenelemente jeweils lokale erste Datenelemente mit gleicher lokaler Identifikationsinformation, aber nur bedingt mit gleicher globaler Indentifikationsinformation.

Bei der Initialisierung des medizinischen Gerätes führt das erfindungsgemäße Programm dazu, daß jeder Rechner bzw. jede Steuereinheit eine Anforderungsliste abarbeitet. In der Anforderungsliste sind die ersten Datenelemente eines Rechners mit ihrer globalen Identifikationsinformation enthalten, die der Rechner von anderen Rechnern aktualisiert beziehen muß. Zusätzlich kann in der Liste die Information enthalten sein, welche Rechner die aktualisierten Daten bereitstellen können. Die Abarbeitung der Anforderungsliste besteht nun darin, an die entsprechenden bereitstellenden Rechner diese Information über die Kommunikationsschnittstellen und das angeschlossene Kommunikationsmedium zu versenden. Die empfangenden Rechner weisen dann den ersten Datenelemente zugeordnete zweite Datenelemente zu ("Send"), in die eingetragen wird, an welchen Rechner neue Daten zu übermitteln sind. Nach der Initialisierungsphase sind damit alle Informationen ausgetauscht, die die Rechner benötigen, um sich im Laufe des Betriebes des Hämodialysegerätes aktualisierte Datenelemente zuzusenden.

Im Beispiel in Fig. 3 hat der Aktionsrechner 200a der Bedieneinheit 20 dem Hilfsrechner 220b der Hydraulikeinheit 22 mitgeteilt, daß er ständig die aktuellen Werte der Dialysierflüssigkeitstemperatur benötigt, deren erstes Datenelement den Namen "H2_PT07Celsius" mit der globalen ID "1001" trägt. In das entsprechende zweite Datenelemente im Speicherelement 222b im Hilfsrechner der Hydraulikeinheit wird daher die Information "200a" für den Aktionsrechner der Bedieneinheit eingetragen.

Der Aktionsrechner 220a der Hydraulikeinheit 22 hat wiederum dem Aktionsrechner 200a der Bedieneinheit 20 mitgeteilt, daß er die aktuellen Werte der Soll-Konzentrationen von Natrium- und Bikarbonationen in der Dialysierflüssigkeit von diesem Rechner beziehen muß. Den ersten Datenelementen mit dem Namen "M1_SollNatrium" und "M1_SollBicarbonat" mit der globalen ID "0" bzw. "1" sind daher im Speicherelement 202a des Aktionsrechners 200a der Bedieneinheit 20 zweite Datenelemente zugeordnet, die jeweils den Wert "220a" für den Aktionsrechner der Hydraullkeinheit als Zielrechner beinhalten.

Die gezeigten ersten Datenelemente des Arbeitsrechners 220a der Hydraulikeinheit 22 im Speicherelement 222a sollen dagegen an keine weiteren Rechner übermittelt werden, wodurch die zweiten Datenelemente entsprechend leer bleiben bzw. mit nicht näher gezeigten Inhalten für diesen Fall versehen werden.

Wird nun im Laufe des Betriebes des Hämodialysegerätes ein erstes Datenelement in irgendeinem der Rechner mit einem neuen Inhalt beschrieben, so wird in dieser Ausführungsform ein dem ersten Datenelement zugeordnetes drittes Datenelement ("Now") in einen ersten Zustand versetzt ("1"). Ansonsten ist das dritte Datenelement in einem zweiten Zustand ("0").

Bezogen auf das Gerät nach Fig. 2 hat der Bediener beispielsweise einen neuen Sollwert für die Natriumionenkonzentration M1_SollNatrium" über den Touchscreen 34 der Bedieneinheit 20 eingegeben, wodurch dem entsprechenden ersten Datenelement im Speicherelement 202a ein neuer Wert zugewiesen wurde. Durch die Zuordnungsfunktion ist das entsprechende dritte Datenelement von dem zweiten Zustand in den ersten Zustand überführt worden. Der Sollwert für die Bikarbonationenkonzentration ist dagegen unverändert geblieben, wodurch das zugehörige dritte Datenelement im zweiten Zustand verblieben ist.

Am Ende eines bestimmten Programmzyklus, d.h. in regelmäßigen Abständen sieht das erfindungsgemäße Programm vor, alle ersten Datenelemente bzgl. des Status des dritten Datenelementes zu überprüfen. Falls das dritte Datenelemente im ersten Zustand ist, wird das erste Datenelement zusammen mit der globalen Identifikationsinformation an den Rechner gesendet, der im zweiten Datenelement genannt ist. Nach der in Fig. 3 gezeigten Situation wird daher lediglich das erste Datenelement mit der globalen ID "0", der Sollwert für die Natriumionenkonzentration, von dem Aktionsrechner 200a der Bedieneinheit 20 an den Aktionsrechner 220a der Hydraulikeinheit 22 übermittelt.

An dieser Stelle sei darauf hingewiesen, daß der Einsatz der dritten Datenelemente nur eine mögliche Ausführungsform darstellt. Das in Fig. 3 gezeigt Beispiel läßt sich leicht auf den in Fig. 1 gezeigten Ablauf übertragen.

Durch die Rechnereinheiten mit dem erfindungsgemäßen Mikroprozessorprogramm wird letztendlich sichergestellt, daß alle Rechner des medizinischen Gerätes ständig über die gleichen aktualisierten ersten Datenelemente verfügen, ohne daß es der individuellen Anweisung an bestimmten Programmstellen zur Übertragung von Datenelementen bedarf. Des weiteren ist es nicht notwendig, an einer Stelle des Systems die Gesamtheit der ersten Datenelemente physikalisch vorzuhalten ("Virtuelle Gesamt DB").

Die Implementierung des erfindungsgemäßen Programms gemäß des kennzeichnenden Teils des Anspruchs 1 läßt sich mit Hilfe spezieller Programmierwerkzeuge leicht und übersichtlich durchführen. Mit Hilfe eines solchen Werkzeuges kann die gesamte erforderliche Information mit Hilfe von Tabellen ähnlich wie in Fig. 3 gepflegt werden und automatisiert in die Anforderungslisten oder entsprechende Programmteile eingebracht werden.

## Patentansprüche

1. Hämodialyse- und/oder -filtrationsgerät, welches eine Blutbehandlungskomponente und eine Blutleitung zum Transport von Blut zwischen einem Patienten und der Blutbehandlungskomponente aufweist, mit automatisierter Datenaktualisierung
mit mindestens zwei Rechnereinheiten
wobei die mindestens zwei Rechnereinheiten je mindestens einen Mikroprozessor, eine Speichereinheit sowie eine Kommunikationsschnittstelle aufweisen,
wobei die Kommunikationsschnittstellen der mindestens zwei Rechnereinheiten über ein Kommunikationsmedium zum Datenaustausch miteinander verbunden sind,
**dadurch gekennzeichnet**
**dass** in einer ersten Speichereinheit der mindestens zwei Rechnereinheiten ein Programm zur Ausführung durch den Mikroprozessor hinterlegt ist, wonach in regelmäßigen Abständen erste Datenelemente in der ersten Speichereinheit bei einer Wertzuweisung dahingehend durch den Mikroprozessor mittels des Programms überprüfbar sind, ob eine Versendung über das Kommunikationsmedium an eine weitere der mindestens zwei Rechnereinheiten zur Aktualisierung der Datenelemente in deren Speichereinheit vorgesehen ist und diese Übersendung bei positiver Prüfung durchgeführt wird.

2. Hämodialyse- und/oder -filtrationsgerät nach Anspruch 1, **dadurch gekennzeichnet, daß** jedem zum Datenaustausch bestimmten ersten Datenelement in dem entsprechenden Speicherelement eine für die Rechnereinheiten global eindeutige Identifizierungsinformation zugeordnet ist.

3. Hämodialyse- und/oder -filtrationsgerät nach Anspruch 1 oder 2, **dadurch gekennzeichnet, daß** jedem zum Datenaustausch bestimmten ersten Datenelement ein zweites Datenelement zugeordnet ist, das Informationen darüber beinhaltet, an welche Rechnereinheit aktualisierte Datenelemente automatisch übertragen werden sollen.

4. Hämodialyse- und/oder -filtrationsgerät nach einem der vorangegangenen Ansprüche, **dadurch gekennzeichnet, daß** die Kommunikationsschnittstellen CAN-Bus Schnittstellen sind.

5. Hämodialyse- und/oder -filtrationsgerät nach einem der vorangegangenen Ansprüche, **dadurch gekennzeichnet, daß** die Überprüfung zur Versendung aktualisierter erster Datenelemente überprüft, ob sich das erste Datenelement gegenüber der vorangegangen Überprüfung verändert hat.

6. Hämodialyse- und/oder -filtrationsgerät nach Anspruch 5, **dadurch gekennzeichnet, daß** den ersten Datenelementen dritte Datenelemente zugeordnet sind, die die Daten der ersten Datenelemente im vorangegangenen Zyklus beinhalten, damit die Überprüfung anhand eines Vergleiches der ersten und dritten Datenelemente durchgeführt werden kann.

7. Hämodialyse- und/oder -filtrationsgerät nach Anspruch 5, **dadurch gekennzeichnet, daß** den ersten Datenelementen dritte Datenelemente zugeordnet sind, die bei einer anstehenden Übertragung einen ersten Zustand und bei keiner anstehenden Übertragung einen zweiten Zustand aufweisen.

8. Hämodialyse- und/oder -filtrationsgerät nach Anspruch 7, **dadurch gekennzeichnet, daß** der erste Zustand automatisch dann zugewiesen wird, wenn dem entsprechendem ersten Datenelement ein Wert zugewiesen wird.

9. Hämodialyse- und/oder -filtrationsgerät nach Anspruch 1, **dadurch gekennzeichnet, daß** das Hämodialyse- und/oder -filtrationsgerät eine Bedieneinheit als erste Steuereinheit, eine Hydraulikeinheit als zweite Steuereinheit und eine Funktionseinheit als dritte Steuereinheit ausweist, wobei alle drei Steuereinheiten je einen Aktionsrechner und einen Hilfsrechner als Rechnereinheiten beinhalten, die Eigenschaften nach Anspruch 1 zur gegenseitigen Aktualisierung ihrer Daten aufweisen.

## Claims

1. A haemodialysis and/or haemofiltration unit which has a blood treatment component and a blood line to transport blood between a patient and the blood treatment component having automated data updating
using at least two processor units,
wherein the at least two processor units each have at least one microprocessor, a memory unit as well as a communication interface;
wherein the communication interfaces of the at least two processor units are connected to one another via a communication medium for data exchange,
**characterised in that**
a program for carrying out by the microprocessor is stored on a first memory unit of the at least two processor units, according to which program first data elements in the first memory unit can be checked at regular intervals by the microprocessor by means of the program on a value allocation as to whether a transmission via the communication medium to a further one of the at least two processor units is provided for updating the data elements in their memory unit and whether this transmission is carried out on a positive check.

2. A haemodialysis and/or haemofiltration unit in accordance with claim 1, **characterised in that** identification information which is globally unambiguous for the processor units is associated with each first data element in the corresponding memory element intended for data exchange.

3. A haemodialysis and/or haemofiltration unit in accordance with claim 1 or claim 2, **characterised in that** a second data element is associated with each first data element intended for data exchange, said second data element containing information as to the processor unit to which updated data elements should be transmitted automatically.

4. A haemodialysis and/or haemofiltration unit in accordance with one of the preceding claims, **characterised in that** the communication interfaces are CAN bus interfaces.

5. A haemodialysis and/or haemofiltration unit in accordance with one of the preceding claims, **characterised in that** the check for the transmission of updated first data element checks whether the first data element has changed with respect to the preceding check.

6. A haemodialysis and/or haemofiltration unit in accordance with claim 5, **characterised in that** third data elements are associated with the first data elements, said third data elements containing the data of the first data elements in the preceding cycle so that the check can be carried out with reference to a comparison of the first and third data elements.

7. A haemodialysis and/or haemofiltration unit in accordance with claim 5, **characterised in that** third data elements are associated with the first data elements, said third data elements having a first state on an upcoming transmission and a second state when no transmission is upcoming.

8. A haemodialysis and/or haemofiltration unit in accordance with claim 7, **characterised in that** the first state is automatically assigned when a value is allocated to the corresponding first data element.

9. A haemodialysis and/or haemofiltration unit in accordance with claim 1, **characterised in that** the haemodialysis and/or haemofiltration unit has an operating unit as a first control unit, a hydraulic unit as a second control unit and a function unit as a third control unit, wherein all three control units each contain an action processor and an auxiliary processor as processor units which have properties in accordance with claim 1 for the mutual updating of their data.

## Revendications

1. Appareil d'hémodialyse et/ou de filtration, lequel présente un composant de traitement du sang et une conduite de sang pour le transport de sang entre un patient et le composant de traitement du sang, comprenant une actualisation automatisée des données
avec au moins deux unités informatiques
les au moins deux unités informatiques présentant respectivement au moins un microprocesseur, une unité de mémoire ainsi qu'une interface de communication,
les interfaces de communication des au moins deux unités informatiques étant reliées l'une à l'autre par le biais d'un moyen de communication pour l'échange de données,
caractérisé en ce
dans une première unité de mémoire des au moins deux unités informatiques, est enregistré un programme pour l'exécution par le microprocesseur, selon lequel des premiers éléments de donnée dans la première unité de mémoire peuvent être vérifiés à intervalles réguliers par le microprocesseur au moyen du programme lors d'une affectation de valeurs, pour savoir si un envoi par le biais du moyen de communication à une autre des au moins deux unités informatiques est prévu pour l'actualisation des éléments de donnée dans leur unité de mémoire et cette transmission est exécutée en cas de vérification positive.

2. Appareil d'hémodialyse et/ou de filtration selon la revendication 1, **caractérisé en ce qu'**une information d'identification globalement univoque pour les unités informatiques est attribuée à chaque premier élément de donnée destiné à l'échange de données dans l'élément de mémoire correspondant.

3. Appareil d'hémodialyse et/ou de filtration selon la revendication 1 ou 2, **caractérisé en ce qu'**un second élément de donnée, qui comprend des informations indiquant à quelle unité informatique des éléments de donnée actualisés doivent être transmis automatiquement, est attribué à chaque premier élément de donnée destiné à l'échange de données.

4. Appareil d'hémodialyse et/ou de filtration selon l'une des revendications précédentes, **caractérisé en ce que** les interfaces de communication sont des interfaces de bus CAN.

5. Appareil d'hémodialyse et/ou de filtration selon l'une des revendications précédentes, **caractérisé en ce que** la vérification pour l'envoi de premiers éléments de donnée actualisés vérifie si le premier élément de donnée a changé par rapport à la vérification précédente.

6. Appareil d'hémodialyse et/ou de filtration selon la revendication 5, **caractérisé en ce que** des troisièmes éléments de donnée, qui comprennent les données des premiers éléments de donnée dans le cycle précédent, sont attribués aux premiers éléments de donnée pour que la vérification puisse être exécutée au moyen d'une comparaison des premiers et troisièmes éléments de donnée.

7. Appareil d'hémodialyse et/ou de filtration selon la revendication 5, **caractérisé en ce que** des troisièmes éléments de donnée, qui présentent un premier état lors d'une transmission en suspens et un second état s'il n'y a pas de transmission en suspens, sont attribués aux premiers éléments de donnée.

8. Appareil d'hémodialyse et/ou de filtration selon la revendication 7, **caractérisé en ce que** le premier état est affecté automatiquement quand une valeur est affectée au premier élément de donnée correspondant.

9. Appareil d'hémodialyse et/ou de filtration selon la revendication 1, **caractérisé en ce que** l'appareil d'hémodialyse et/ou de filtration présente une unité de commande en tant que première unité de contrôle, une unité hydraulique en tant que seconde unité de contrôle et une unité fonctionnelle en tant que troisième unité de contrôle, les trois unités de contrôle comprenant respectivement un ordinateur d'action et un ordinateur auxiliaire en tant qu'unités informatiques, qui présentent des caractéristiques selon la revendication 1 pour l'actualisation réciproque de leurs données.
